# FASCICULE DE BREVET EUROPEEN

(11) **EP 0 820 763 B1**
(45) Date de publication et mention de la délivrance du brevet: **13.09.2000**
(21) Numéro de dépôt: 97401234.6
(22) Date de dépôt: 03.06.1997
(51) Int. Cl.: A61K 7/48

(54) **Utilisation de dérivés de l'acide N,N'-dibenzyl éthylènediamine comme agents dépigmentants**
Verwendung von N,N'-Dibenzyl-Äthylendiaminsäure-Derivaten als Depigmentierungsagentien
Use of N,N'-dibenzyl-ethylene diamine acid derivatives as depigmentation agents

(30) Priorité: 26.07.1996 FR 9609459
(43) Date de publication de la demande: 28.01.1998
(73) Titulaire: L'OREAL, 75008 Paris (FR)
(72) Inventeur: Galey, Jean-Baptiste, 93600 Aulnay-Sous-Bois (FR); Marrot, Laurent, 93190 Livry Gargan (FR); Causse, Catherine, 92340 Bourg La Reine (FR)
(74) Mandataire: Lhoste, Catherine

(56) Documents cités:
- WO-A-94/11338

## Description

La présente invention se rapporte à l'utilisation d'un dérivé de l'acide N,N'-dibenzyl éthylènediamine N,N'-diacétique dans une composition à application topique, comme agent dépigmentant et/ou blanchissant de la peau humaine.

La couleur de la peau est fonction de différents facteurs et notamment des saisons de l'année, de la race et du sexe, et elle est principalement déterminée par la concentration de mélanine produite par les mélanocytes. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

Depuis plusieurs années, on cherche à diminuer et/ou ralentir la production de mélanine en vue de dépigmenter ou blanchir la peau en agissant sur un ou plusieurs des points de la synthèse biochimique intracellulaire de la mélanine.

Aussi depuis un bon nombre d'années, différentes molécules ont déjà été utilisées et testées comme agent dépigmentant ou blanchissant.

Le mécanisme de formation de la pigmentation de la peau, c'est-à-dire de la formation de la mélanine est particulièrement complexe et fait intervenir schématiquement les principales étapes suivantes :
Tyrosine → Dopa → Dopaquinone → Dopachrome → Mélanine

La tyrosinase est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la viabilité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants dans des compositions sont notamment des composés tels que la vitamine C, les dérivés de vitamine C ou de vitamine E, l'arbutine, l'hydroquinone, l'acide kojique, les dérivés placentaires, le glutathion et ses dérivés.

Ces différents composés sont connus pour agir sur la synthèse et/ou l'activité de la tyrosinase, enzyme qui entre en jeu dans la synthèse de la mélanine, ou pour réduire la quantité de mélanine formée ou encore pour stimuler l'élimination de la mélanine au travers des kératinocytes. Malheureusement, ils sont soit toxiques, cas de l'hydroquinone, soit instables en solution, cas de la vitamine C et de l'acide kojique ce qui complique quelque peu la fabrication de la composition, soit encore peuvent présenter des odeurs désagréables et notamment des odeurs soufrées en ce qui concerne le glutathion, ce qui en limite par conséquent l'utilisation. De plus le nombre de ces composés est très limité.

Aussi, il subsiste le besoin d'un nouvel agent blanchissant de la peau, à action aussi efficace que ceux connus mais n'ayant pas leurs inconvénients, c'est-à-dire qui soit stable dans une composition, non toxique pour la peau et qui ne présente pas d'odeur désagréable et ce surtout dans la mesure où cet agent est appliqué sur la peau.

La demanderesse a trouvé de manière inattendue que certains chélateurs de fer présentaient la propriété d'inhiber la synthèse de la mélanine et étaient susceptibles d'agir ainsi sur la pigmentation et les taches de la peau sans toxicité.

Certes, l'utilisation de chélateurs du cuivre comme agents dépigmentants est connue de longue date. En effet, ces chélateurs agissent sur les ions cuivre présents au site actif de la tyrosinase, enzyme clé de la mélanogénèse. A titre d'exemple, il a été reporté que la L-mimosine, qui est une hydroxypyridinone naturelle formant des complexes très stables avec le cuivre, est un bon inhibiteur de la tyrosinase (Hider et coll, Biochem. J. 1989, 257:289), comme l'acide kojique qui interagit également fortement avec le cuivre. Toutefois, ces chélateurs présentent l'inconvénient d'être toxiques, surtout la L-mimosine, et de présenter des effets secondaires liés à l'interférence avec le métabolisme de certains métaux.

En revanche, l'utilisation de chélateurs du fer comme agents dépigmentants a été beaucoup moins étudiée. Certains chélateurs sont décrits comme capables de dépigmenter la peau du vison (Blumenkrantz et coll, Acta Agric. Scand. 1987, 37:375). Toutefois, les composés décrits dans ce document présentent une certaine toxicité qui rend impossible leur utilisation topique sur la peau humaine.

En outre, des hydrolysats de lactoferrine ont été brevetés comme agents dépigmentants (Morinaga, JP-A-04059714 et EP-A-438750). Toutefois, on cherche à éviter l'utilisation de produits d'origine animale.

Par ailleurs, les composés connus comme chélateurs de fer et couramment utilisés dans le domaine cosmétique ou dermatologique n'ont jamais été jusqu'à présent utilisés pour dépigmenter ou blanchir la peau.

En particulier, certains N,N'-diarylméthylène éthylènediaminacétates, dont l'acide N,N'-di-(3-(hydroxybenzyle)-éthylènediamine N,N'-diacétique, ont été décrits dans WO 94/11338 comme actifs dans une composition cosmétique destinée à protéger la peau contre le stress oxydatif dû à la formation de radicaux libres.

Le composé selon l'invention répond à la formule générale (II) suivante :

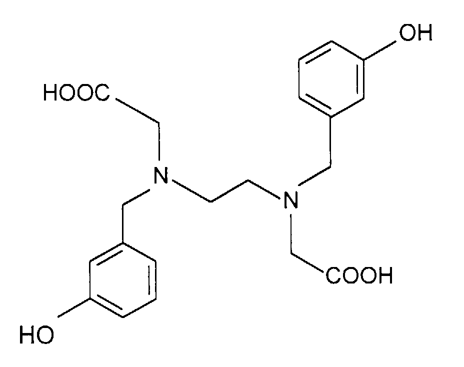

Le composé de l'invention peut être préparé comme décrit dans le document WO 94/11338 déposé au nom de la demanderesse.

Le composé de formule (II) est l'acide N,N'-di-(3-hydroxybenzyle)-éthylènediamine N,N'-diacétique.

La demanderesse a trouvé de façon surprenante que ce composé avait l'avantage de posséder une activité dépigmentante importante tout en n'en présentant pas les inconvénients des composés utilisés antérieurement.

L'invention a donc pour objet l'utilisation du composé de formule (II) comme agent dépigmentant et/ou blanchissant de la peau humaine dans une composition cosmétique.

L'invention a encore pour objet un procédé cosmétique de dépigmentation et/ou blanchiment de la peau humaine, caractérisé en ce qu'il consiste à appliquer sur la peau tachée, le composé de formule (II) dans un milieu cosmétiquement acceptable.

Un test a mis en évidence l'activité du composé selon l'invention comme inhibiteur de la tyrosinase par évaluation de son effet sur l'activité dopa-oxydase de la tyrosinase des mélanocytes humains. Le composé testé est l'acide N,N'-di-(3-hydroxybenzyle)-éthylènediamine N,N'-diacétique de la formule (II) :

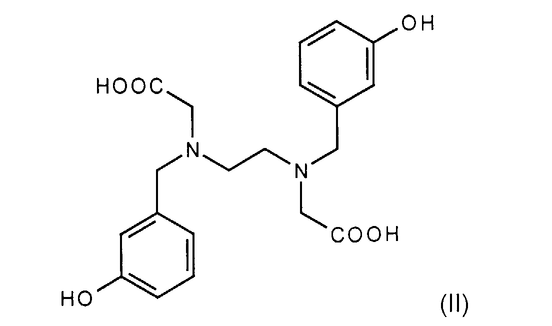

Des mélanocytes humains ont été préparés et mis en culture, puis introduits dans des milieux comportant différentes concentrations non cytotoxiques du composé de formule (II) (0,1 mM, 0,25 mM, 0,5 mM et 0,75 mM).

Après trois jours, les mélanocytes ont été « trypsinisés » dans un mélange de trypsine / EDTA 0,05 % / 0,02 % dans un tampon phosphate (50 mM à pH 6,8), lavés à trois reprises dans le tampon phosphate contenant 1 % (w/v) de Triton x-100 et soumis à des ultrasons. Après centrifugation, des échantillons ont été prélevés et mélangés avec de la L-DOPA 5 mM dans du tampon phosphate. L'activité de la tyrosinase a été évaluée par spectrophotométrie en mesurant l'augmentation de l'absorbance à 475 nm due à la formation de dopachrome à partir de DOPA 5 mM. La lecture de l'absorbance a été effectuée sur un lecteur de microplaques à 25°C pendant 60 mn.

L'activité de la tyrosinase cellulaire est mesurée par rapport à une gamme étalon réalisée avec la tyrosinase commerciale.

Les résultats ont été les suivants :

| | | | | | |
|---|---|---|---|---|---|
| Concentration en composé de formule (II) | 0 | 0,1 mM | 0,25 mM | 0,5 mM | 0,75 mM |
| Pourcentage d'inhibition | 0 | 19 % | 30 % | 33 % | 42 % |

Ces résultats montrent que le composé selon l'invention présente une bonne inhibition de la tyrosinase et donc de la production de mélanine.

Le composé de formule (II) peut être présent dans la composition selon l'invention en une quantité allant de 0,01 à 10 % et de préférence de 0,1 à 5 % du poids total de la composition.

La composition contenant le composé selon l'invention comporte un milieu physiologiquement acceptable, c'est-à-dire compatible avec la peau, le cuir chevelu et les cheveux, et constitue plus particulièrement une composition à application topique, cosmétique et/ou dermatologique, notamment blanchissante et/ou dépigmentante.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

La composition selon l'invention peut comprendre tous les ingrédients classiquement utilisés dans le domaine cosmétique ou dermatologique, dans des concentrations habituelles. Ces ingrédients sont en particulier choisis parmi les corps gras, les conservateurs, les vitamines, les gélifiants, les parfums, les tensioactifs, l'eau, les antioxydants, les charges, les hydratants, les filtres et leurs mélanges.

Comme corps gras utilisables dans l'invention, on peut citer les huiles minérales (huile de vaseline), les huiles d'origine végétale (huile de jojoba), les huiles d'origine animale, les huiles de synthèse (palmitate d'isopropyle), les huiles siliconées (cyclopentadiméthylsiloxane) et les huiles fluorées. On peut aussi utiliser des alcools gras (alcool stéarylique), des acides gras, des cires.

Comme tensioactifs utilisables dans l'invention, on peut citer par exemple les esters d'acide gras et de polyéthylène glycol tels que le stéarate de polyéthylèneglycol, et les esters d'acide gras tels que le stéarate de sodium.

L'invention va maintenant être illustrée à l'aide des exemples qui suivent. Les concentration sont données en pourcentage en poids.

### Exemple 1: crème blanchissante pour le visage

- acide N,N'-di(3-hydroxybenzyle) ethylènediamine N,N'-diacétique 1 %
- stéarate de sodium 3 %
- huile de vaseline 6 %
- conservateur 1 %
- cyclopentadiméthylsiloxane 2 %
- alcool stéarylique 1 %
- parfum 1 %
- eau qsp 100 %

La crème obtenue utilisée en application quotidienne, permet d'obtenir un blanchiment de la peau.

### Exemple 2: crème blanchissante pour les mains

- l'acide N,N'-di(3-hydroxybenzyle) ethylènediamine N,N'-diacétique 3 %
- huile de jojoba 13 %
- cire de sipol 6 %
- palmitate d'isopropyle 2 %
- stéarate de polyéthylène glycol 3 %
- glycérol (hydratant) 15 %
- conservateur 0.5 %
- parfum 1 %
- eau qsp 100 %

La crème obtenue peut être utilisée quotidiennement et est apte à atténuer voire effacer les taches sur les mains.

## Revendications

1. Utilisation cosmétique du composé de formule (II) : 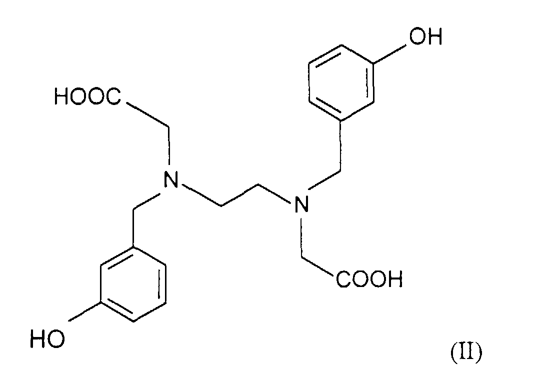 comme agent dépigmentant et/ou blanchissant de la peau humaine dans une composition cosmétique.

2. Utilisation selon la revendication 1, caractérisée en ce que ledit composé est présent en une quantité allant de 0,01 à 10 % du poids total de la composition.

3. Utilisation selon l'une quelconque des revendications 1 ou 2, caractérisée en ce que la composition comprend, en outre, au moins un ingrédient choisi parmi les corps gras, les conservateurs, les vitamines, les gélifiants, les parfums, les tensioactifs, l'eau, les antioxydants, les charges, les filtres, les hydratants et leurs mélanges.

4. Utilisation selon la revendication précédente, caractérisée en ce que le corps gras est choisi parmi les huiles et les cires.

5. Procédé cosmétique de dépigmentation et/ou blanchiment de la peau humaine, caractérisé en ce qu'il consiste à appliquer sur la peau tachée le composé de formule (II): 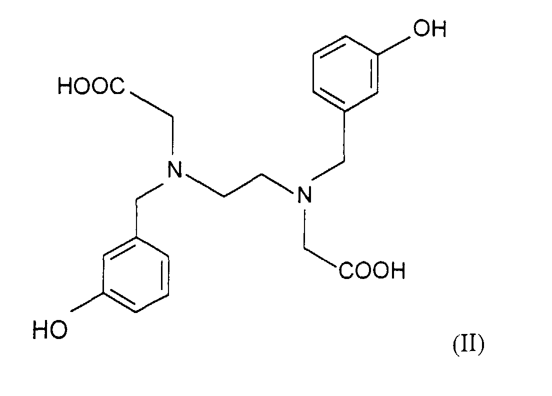 dans un milieu cosmétiquement acceptable.

6. Procédé selon la revendication précédente, caractérisé en ce que ledit composé est présent en une quantité allant de 0,01 à 10 % du poids total de la composition.

## Patentansprüche

1. Kosmetische Verwendung der Verbindung der Formel (II): 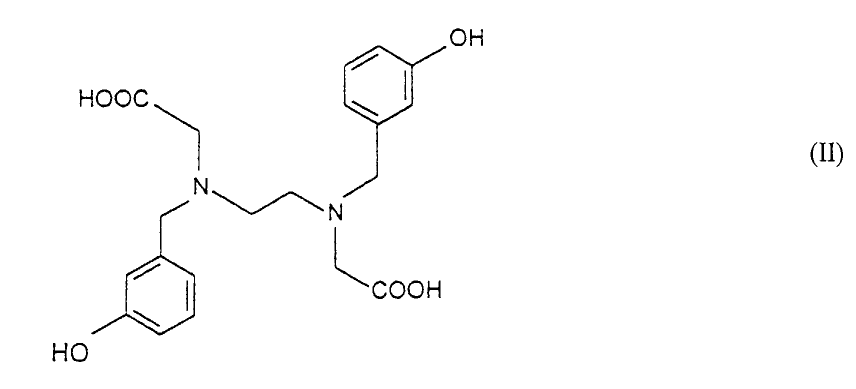 als Mittel zur Depigmentierung und/oder Bleichung der menschlichen Haut in einer kosmetischen Zusammensetzung

2. Verwendung nach Anspruch 1, dadurch gekennzeichnet, daß die Verbindung in einem Mengenanteil von 0,01 bis 10 % des Gesamtgewichts der Zusammensetzung vorliegt.

3. Verwendung nach einem der Ansprüche 1 oder 2, dadurch gekennzeichnet, daß die Zusammensetzung ferner mindestens einen Bestandteil enthält, der unter den Fettsubstanzen, Konservierungsmitteln, Vitaminen, Gelbildnern, Parfums, grenzflächenaktiven Stoffen, Wasser, Antioxidantien, Füllstoffen, Filtern, Hydratisierungsmitteln und deren Gemischen ausgewählt ist.

4. Verwendung nach dem vorhergehenden Anspruch, dadurch gekennzeichnet, daß die Fettsubstanz unter den Ölen und Wachsen ausgewählt ist.

5. Kosmetisches Verfahren zur Depigmentierung und/oder Bleichung der menschlichen Haut, dadurch gekennzeichnet, daß es darin besteht, in einem kosmetisch akzeptablen Medium die Verbindung der Formel (II) auf die Haut, die Pigmentflecken aufweist, aufzutragen:

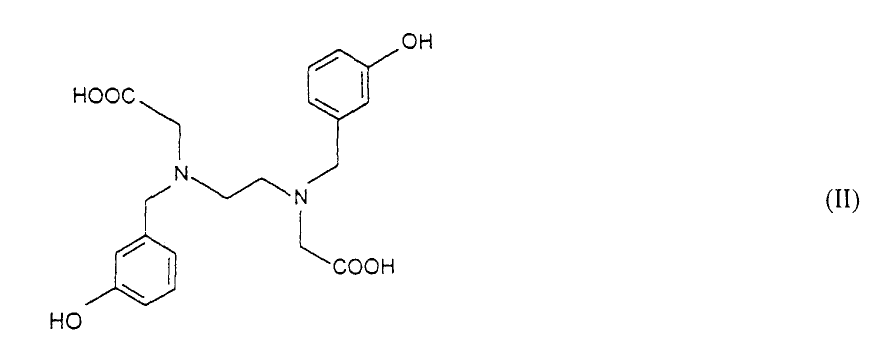

6. Verfahren nach dem vorhergehenden Anspruch, dadurch gekennzeichnet, daß die Verbindung in einem Mengenanteil von 0,01 bis 10 % des Gesamtgewichts der Zusammensetzung vorliegt.

## Claims

1. Cosmetic use of the compound of formula (II): 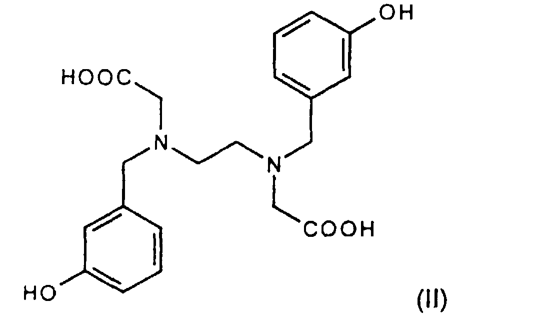 as a depigmenting and/or bleaching agent for human skin in a cosmetic composition.

2. Use according to Claim 1 characterized in that the said compound is present in an amount ranging from 0.01 to 10 % of the total weight of the composition.

3. Use according to either of Claims 1 and 2, characterized in that the composition also comprises at least one ingredient chosen from fatty substances, preserving agents, vitamins, gelling agents, fragrances, surfactants, water, antioxidants, fillers, screening agents, hydrating agents and mixtures thereof.

4. Use according to the preceding claim, characterized in that the fatty substance is chosen from oils and waxes.

5. Cosmetic process for the depigmentation and/or bleaching of human skin, characterized in that it consists of applying to blemished skin the compound of formula (II): 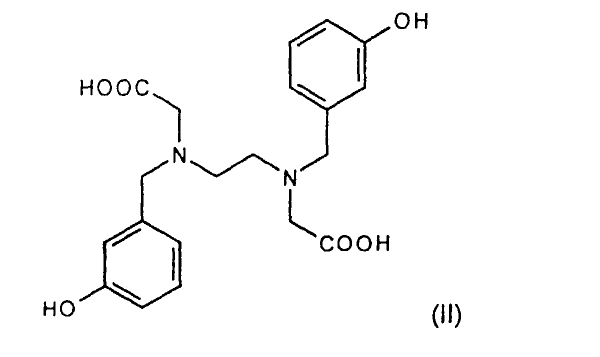 in a cosmetically acceptable medium.

6. Process according to the preceding claim, characterized in that the said compound is present in an amount ranging from 0.01 to 10 % of the total weight of the composition.
